# EUROPEAN PATENT SPECIFICATION

(11) **EP 2 312 952 B1**
(45) Date of publication and mention of the grant of the patent: **11.11.2015**
(21) Application number: 09794732.9
(22) Date of filing: 02.07.2009
(51) Int. Cl.: A01N 47/44, A01N 31/02, A01P 1/00, A61K 31/047, A61K 31/155, A61K 8/34, A61K 8/43, A61L 15/20, A61L 15/46, A61P 31/02, A61P 31/04, A61Q 17/00

(54) **ANTIMICROBIAL COMPOSITION**
ANTIMIKROBIELLE ZUSAMMENSETZUNG
COMPOSITION ANTIMICROBIENNE

(30) Priority: 07.07.2008 SE 0801639
(43) Date of publication of application: 27.04.2011
(73) Proprietor: Ambria Dermatology AB, 566 33 Habo (SE)
(72) Inventor: FAERGEMANN, Jan, S-413 19 Göteborg (SE)
(74) Representative: Ström & Gulliksson AB
(86) International application number: PCT/SE2009/050854
(87) International publication number: WO 2010/005378

(56) References cited:
- WO-A1-97/32479
- WO-A1-2007/063065
- WO-A1-2007/063065
- TAWIL G G ET AL: "Potentiation of Chlorhexidine by Propylen Glycol", ALEXANDRIA JOURNAL OF PHARMACEUTICAL SCIENCES, ALEXANDRIA, EG, vol. 2, no. 1, 1 March 1988 (1988-03-01), pages 58-60, XP003026052, ISSN: 1110-1792
- Anonymous: "GNPD - Eye Cream", , 1 June 2008 (2008-06-01), XP55088975, Retrieved from the Internet: URL:http://www.gnpd.com/sinatra/recordpage /924824/from_search/OHDAPuk8vT/ [retrieved on 2013-11-19]
- Anonymous: "GNPD - Soin Hydratant Oxygénant Cream", , 1 December 2003 (2003-12-01), XP055088976, Retrieved from the Internet: URL:http://www.gnpd.com/sinatra/recordpage /242118/from_search/a6VfP8epb1/with_sort/2 / [retrieved on 2013-11-19]
- Anonymous: "GNPD - Moisture Cream SPF 15", , 1 January 2007 (2007-01-01), XP055088977, Retrieved from the Internet: URL:http://www.gnpd.com/sinatra/recordpage /636103/from_search/a6VfP8epb1/with_sort/2 / [retrieved on 2013-11-19]
- Anonymous: "GNPD - Special Trial Set II", , 1 October 2005 (2005-10-01), XP055088979, Retrieved from the Internet: URL:http://www.gnpd.com/sinatra/recordpage /405108/from_search/aV1x6hXooC/with_sort/2 / [retrieved on 2013-11-19]
- TAWIL, G.G. ET AL: 'Potentiation of Chlorhexidine by Propylen Glycol' ALEXANDRIA JOURNAL OF PHARMACEUTICAL SCIENCES vol. 2, no. 1, March 1988, pages 58 - 60, XP003026052

## Description

### Field of the invention

Embodiments of the present invention relates to an antimicrobial composition, comprising between 0.000001 and 5% of chlorhexidine and between 1 and 75% of pentane-1,5-diol and further to the use of such a composition to combat microorganisms.

### Background

Microorganisms may be combated in different ways depending on the reasons for combating them. The approach is different depending on if the purpose is to prevent growth of microorganisms, inhibit further growth or reduce and eliminate the microorganism. Additionally the approach is different depending on where the microorganism is located such as in a liquid, on a surface, within a mammalian body etc.

US 2004/0191274 A1 discloses topical compositions, which may be used as an antiseptic and/or disinfectant. The topical composition disclosed comprises at least one C1 to C4 alcohol; at least one anti-microbial, such as chlorhexidine; and at least one emollient, such as polyethylene glycol.

CN 1130020, Tawil, G.G. et al Alexandria Journal of Pharmaceutical Sciences, 1988, 2(1), 1 March, 58-60 and Frank M. von et al Pharmaceutische Industrie, 1991, 53(5) 512-516 all disclose combinations of chlorhexidine and propyleneglycol. Frank M. von et al concludes that no synergy can be found between chlorhexidine and propyleneglycol in combination. On the contrary, previous findings of Tawil, G.G. et al states the propyleneglycol should potentiate the bactericidal effect of chlorhexidine. It is shown that an antimicrobial compositions comprising chlorhexidine acetate and propylene glycol, the propylene glycol potentiates the antimicrobial effect of chlorhexidine. However, Tawil, G.G. et al present no data for propyleneglycol when tested alone. JP 2003267862 discloses a combination of chlorhexidine and 1,3-butandiol.

WO 07/063065 discloses the use of a composition comprising pentane-1,5-diol to reduce and/or eliminate odour from a mammal, such as odour from urine, menstruation, faeces, liquids from leg ulcers, decubital ulcers, blood and perspiration. Furthermore, WO 07/063065 discloses an absorption product comprising pentane-1,5 diol to reduce and/or eliminate odour from a mammal, such as odour from urine, menstruation, faeces, liquids from leg ulcers, decubital ulcers, blood, and perspiration.

There are various compositions and compounds, which are known to possess anti-bacterial properties.

WO 97/32479 A1 describes the antimicrobic properties of bacteriostatic type of chlorobutanol. Furthermore, it discloses that the association of chlorhexidine and chlorobutanol in anhydrous or hemihydrate form potentiates the antiseptic effect of the two. This potentiation was shown to work on both gram-negative and gram-positive bacteria.

One example of a compound known to possess anti-bacterial properties is propane-1,2-diol (propylene glycol), which is a diol widely used to combat microorganisms in dermatology. Although not as widely used as propylene glycol, also pentane-1,5-diol have been used to combat microorganisms in dermatology.

According to WO 04/112765, the antibacterial effect of pentane-1,5-diol is believed to be shared by other low-molecular weight aliphatic diols Further, use of pentane-1,5-diol is disclosed to have effect against multiresistant strains of bacteria.

Chlorhexidine is a chemical antiseptic. It is bactericidal to both gram-positive and gram-negative microbes. Further, it is bacteriostatic. The mechanism of action is believed to be membrane disruption, and not ATPase inactivation as previously thought. Chlorhexidine is used for general skin cleansing, such as in surgical scrubs for pre-operative skin treatment. It has a long duration of action and a low toxicity. Skin sensitivity to chlorhexidine has been reported. Also, chlorhexidine may be neurotoxic at high levels, whereby it would be beneficial to decrease the level of chlorhexidine, while still maintaining its effect.

Multi-resistance of bacteria to antibiotics is becoming more and more common. In the health sector there is today a mounting concern worldwide regarding the future use of traditional antimicrobials. Alternative methods and approaches have to be used to manufacture affective antimicrobial agents.

Accordingly, there is an increasing medical need to identify new antimicrobial compositions, which can be effectively used to inactivate microorganisms. In particular there is a growing list of microorganisms, such as bacteria, virus and fungi, which become resistant to antibiotics. Additionally, there is an increasing population of individuals becoming allergenic against a variety of antibiotics or preservative components used in antibiotic preparations, leading to needs for novel compositions which can be used as alternatives to conventional technologies.

In addition to finding novel antibiotics, it would also be highly desirable to identify novel synergistic mixtures of known antimicrobial compounds. Several known antibiotics may give rise to adverse side effects at doses applied when administered alone.

### Summary

Accordingly, the present invention seeks to mitigate, alleviate, circumvent or eliminate one or more of the above-identified deficiencies by providing a composition comprising between 0.000001 and 5 wt % chlorhexidine and between 1 and 75 wt % pentane-1,5-diol. Further, various embodiments of the present invention relates to the use of said composition for inactivating microorganisms selected from the group consisting of gram positive and gram negative bacteria, fungi, including yeasts, moulds and dermatophytes and virus, and especially *Staphylococcus aureus* and *P. acnes*.

Advantageous features of the invention are defined in the dependent claims.

### Detailed description of example embodiments

In the context of the present description and the appended claims, chlorhexidine is intended to include various forms of chlorhexidine. Such forms include, but are not limited to chlorhexidine in its free from, salts of chlorhexidine, such as the dihydrochloride, the diacetate or the D-diglyconate, solvates of chlorhexidine and solvated salts of chlorhexidine.

The present invention relates to a composition, such as pharmaceutical composition, comprising between 0.000001 and 5% of chlorhexidine and between 1 and 75% of pentane-1,5-diol

It was envisaged by the present inventor that a synergistic antimicrobial effect is achieved when chlorhexidine is combined with pentane-1,5-diol. Thus, it was envisaged that a composition, comprising both chlorhexidine and pentane-1,5-diol, have higher antimicrobial effect than compositions comprising the same concentrations of either chlorhexidine or pentane-1,5-diol.

Specifically, the synergistic effect between chlorhexidine and pentane-1,5-diol is shown in the examples below. Although, WO 07/063065 discloses that pentane-1,5-diol, when used to reduce odour from a mammal, may be combined with an antiseptic agent, it is silent about any possible synergistic effect. Furthermore, WO 07/063065 is silent about suitable concentrations to be used of said components in a pharmaceutical composition. According to WO 07/063065, at least 0.1 wt% pentane-1,5-diol should be employed. However, as disclosed herein, such a concentration is too low to have any synergistic effect when combined with chlorhexidine.

However, it is not unlikely that a synergistic effect also is obtained by the combination of chlorhexidine and another alkanediol than pentane-1,5-diol, but pentane-1,5-diol is preferred as it is well known with a preferable toxicological profile, low risk for sensibility and skin irritation. Due to the synergistic antimicrobial effect it may be possible to use less of each component and still obtain the desired effect. Further, by using less of each component, each component may give rise to fewer side effects and less severe side effects than used alone. A composition comprising both chlorhexidine and pentane-1,5-diol is more effective to combat microorganisms than a composition only comprising one active component.

Without being bound to any theory it may be assumed that the mechanism of action of chlorhexidine, i.e. the disruption of lipid membrane, is aided by the specific hydrophobicity of pentan-1,5-diol, which appears to be significantly suitable in this respect. Further, the solubilizing properties of pentan-1,5-diol may be important in this context. Alternatively, the antimicrobial effect of pentan-1,5-diol is improved by the simultaneous disruption of lipid membrane caused by chlorhexidine. Another theory is that the mechanism of action of the antimicrobial effect of pentan-1,5-diol is the dissolution of the lipid membranes, thereby enhancing the ability of chlorhexidine to disrupt said lipid membrane, and vice versa. Perhaps, the dissolution effect and hydrophobicity of pentane-1,5-diol cooperates with chlorhexidine in a way maximizing the synergistic effect there between.

A pharmaceutical composition comprising chlorhexidine and pentane-1,5-diol may be used in therapy. When used in therapy it may be administered topically. Such a topically administered composition may be used to treat various infections, such as infections affecting the skin or mucous membranes. Examples of such skin infections include impetigo, secondary infected atopic dermatitis and other dermatosis with secondary infections. Chlorhexidine and pentane-1,5-diol may also be used to treat fungal skin and mucous membrane infections such as tinea pedis, tine corporis, tinea unguium, cutaneous and mucous membrane Candida infections etc. and viral infections such as herpes infections (e. g. herpes labialis), papilloma and pox skin or mucous membrane infections

A pharmaceutical composition, comprising chlorhexidine and a pentane-1,5-diol, may also be used to inactivate microorganisms selected from the group consisting of gram positive and gram negative bacteria, fungi, including yeasts, moulds and dermatophytes and virus. Examples of such microorganisms include, but are not limited to, *Staphylococcus aureus*, *P. acnes, Streptococci,* gram negative rodes, *Candida albicans, Candida glabrata, Malassezia*, the mould *Aspergillus fumigatus* and the dermatophytes *Trichophyton rubrum, T. mentagrophytes, Epidermophyton floccusum, Microsporum canis,* papilloma virus, herpes virus and pox virus. A composition, comprising chlorhexidine and pentane-1,5-diol, has been shown to be especially effective in affecting, i.e. killing off, combating or preventing, *Staphylococcus aureus* and *P. acnes*, as disclosed in the examples below.

A composition for topical administration may take the form of - or being integrated in - a liquid, semi-liquid or solid disinfectant preparation, a bacteriostatic or bacteriocidal solution, lotion, cream, soap, shampoo, ointment, paste, wet towel, hygiene dish, patch, diaper or similar personal hygiene protection device.

If intended for application to the skin or mucous membranes, the composition according to embodiments of the invention may comprise one or several of tonicity adjustment agent or moisturizing agent, such as sodium chloride, urea and lactic acid, w-absorbing agent, colorant, such as calcium carbonate and zinc oxide, and fragrant, such as an aetheric oil. The composition of the invention may also comprise a cationic, neutral, or anionic detergent, in particular a salt of a fatty acid.

Preferably, a composition comprising chlorhexidine and pentane-1,5-diol, also comprises a carrier. The carrier may be an aqueous carrier or a lipid carrier, which further may comprise a thickening agent. Such thickening agents may be selected from cellulose derivatives, in particular methyl cellulose, hydroxymethyl cellulose, hydroxymethyl-propyl cellulose. According to a further embodiment of the invention, the carrier comprises a salt of a fatty acid.

Another embodiment of the invention relates to pharmaceutical compositions as disclosed above, which further includes one or more pharmaceutically acceptable adjuvants or excipients. Such pharmaceutical compositions may be prepared in a manner known in the art and are sufficiently storage-stable and suitable for administration to humans and animals.

A pharmaceutical composition as disclosed herein to be administered topically may take the form of ointments, lotions, pastes, creams, gels, talc, sprays, solutions and emulsions as readily understood to the one skilled in the art. Such ointments, lotions, creams and gels may comprise excipients, such as animal and vegetable fats, waxes, paraffins, starch, tragacanth, cellulose derivatives, polyethylene glycols, silicons, bentonites, silicic acid, talc and zinc oxide or mixtures of these substances. Talc and sprays may comprise excipients, such as lactose, talc, silica acid, aluminium hydroxide, calcium silicate and polyamide powders or mixtures thereof. Sprays may also comprise propellants such as chloroflourohydrocarbons. Solutions and emulsions may comprise excipients, such as solvents, solubilising agents and emulsifiers such as water, ethyl alcohol, isopropylalcohol, ethylcarbonate, ethyl acetate, benzyl alcohol, benzyl benzoate, propylene alcohol, dimethylformamide, oils such as cottonseed oil, groundnut oil, corn germ oil, olive oil, castor oil and sesame oil, glycerol, glycerol formal, tetrahydrofurfuyl alcohol, polyethylene glycols and fatty acid esters such as sorbitan or mixtures thereof.

A pharmaceutical composition as disclosed herein may be administered to a patient in a pharmaceutically effective dose. By "pharmaceutically effective dose" is meant a dose that is sufficient to produce the desired effects in relation to the condition for which it is administered. The exact dose is dependent on the, activity of the compound, manner of administration, nature and severity of the disorder, age and body weight of the patient, and different doses may be needed. The administration of the dose can be carried out both by single administration in the form of an individual dose unit or by several smaller dose units or by multiple administrations of subdivided doses at specific intervals.

The "patient" for the purposes of the present invention includes both humans and other mammal. Thus the methods are applicable to both human therapy and veterinary applications.

One further embodiment of the present invention relates to a composition, such as a pharmaceutical composition, comprising at least 0.000001 wt%, such as at least 0,00005 wt% chlorhexidine and at least 1 wt% of pentane-1,5-diol.

Another embodiment of the present invention relates to a composition, such as a pharmaceutical composition, comprising 0.000001 wt% to 5 % wt%, such as 0,00005 to 5 wt% or 0,00005 to 1 wt%, chlorhexidine and 1 to 75 wt%, such as 1 to 25 wt%, 2 to 15 wt%, or 3 to 15 wt%, of pentane-1,5-diol.

Another embodiment of the present invention relates to a composition, such as a pharmaceutical composition, comprising at least 0,00001 wt% chlorhexidine and at least 3 wt% of pentane-1,5-diol. Although, lower concentrations may be used, the antimicrobial effect is more pronounced at or above these concentrations.

Another embodiment of the present invention relates to a composition, such as a pharmaceutical composition, comprising at least 0,0001 wt% chlorhexidine and at least 5 wt% of pentane-1,5-diol.

As higher concentrations may increase the risks of side effects, another embodiment of the present invention relates to a composition, such as a pharmaceutical composition, comprising between 0,0001 and 5 wt%, such as 0,001 and 2.5 chlorhexidine and between 3 and 25 wt%, such as between 5 and 15 wt%, of pentane-1,5-diol.

Another embodiment of the present invention relates to a pharmaceutical composition comprising at least 0,001 wt% chlorhexidine and pentane-1,5-diol.

Another embodiment of the present invention relates to a pharmaceutical composition comprising chlorhexidine and at least 2 wt% of pentane-1,5-diol.

Another embodiment of the present invention relates to a pharmaceutical composition comprising chlorhexidine and at least 5 wt% of pentane-1,5-diol.

When combined together, chlorhexidine and pentane-1,5-diol give rise to synergistic antibacterial effects as shown in the examples below. These synergistic effects make the use of lower amounts of the respective compounds possible, while still obtaining the desired effect. A combination of chlorhexidine and pentane-1,5-diol is effective in preventing growth of various microorganisms. Such microorganisms include bacteria, fungi and virus. Such bacteria include, but are not limited to, *S. aureus* and *P. acnes*.

Similarly to chlorhexidine, also a composition comprising chlorhexidine and pentan-1,5-diol may be used to used for general skin cleansing, such as in surgical scrubs for pre-operative skin treatment. The disinfection achieved by use of such a composition will be more effective than if only chlorhexidine is used. Furthermore, the well-known water binding effect of diols make them perfect moisturizers to the skin and will therefore make such a treatment less harsh when applied to the skin.

Furthermore, a composition comprising chlorhexidine and pentan-1,5-diol may be used in other types of disinfections and cleaning. Without limitations such disinfections may include treatment of various surfaces, such as floors, walls, table tops, bench tops, table leafs and surgical instruments. Such disinfections may also be combined with autoclavation or may even replace it. In one embodiment such disinfections includes disinfection of skin but other embodiment relating to disinfection excludes disinfections of parts of a human or animal body. Such disinfections excluding disinfections of parts of a human or animal body is to be distinguished from a therapeutic method.

In an embodiment wherein a composition comprising chlorhexidine and pentan-1,5-diol is used to disinfect and/or clean a surface not part of an animal or human body, the content of pentan-1,5-diol may be 1-75 wt% and the content of chlorhexidine 0.000001 % to 5 wt%.

Although the present invention has been described above with reference to specific illustrative embodiments, it is not intended to be limited to the specific form set forth herein. Rather, the invention is limited only by the accompanying claims and other embodiments than the specific above are equally possible within the scope of these appended claims.

In the claims, the term "comprises/comprising" does not exclude the presence of other species or steps. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality.

### Examples

The examples given below are only intended to further illustrate the invention.

### Example 1, MIC of pentane-1,5-diol and chlorhexidine against S. aureus

The minimal inhibitory concentration (MIC) of pentane-1,5-diol and chlorhexidine against *S. aureus* was determined using bacteria concentrations of 6 x 10⁶ (20 µl applicated onto DST-agar plates). The results are depicted below in table 1 and 2.

**Table 1. MIC of pentane-1,5-diol against S. aureus and P. acnes**

| Pentane-1,5-diol (wt %) | MIC (S. aureus) |
|---|---|
| 5 | +++^{a} |
| 6 | ++ |
| 7 | ++ |
| 8 | + |
| 9 | - |
| 10 | - |
| 20 | - |
| 30 | - |
| Control (no pentane-1,5-diol) | +++ |

| | |
|---|---|
| ^{a}: +++: Same growth as on the control plate without any chlorhexidine and/or pentane-1,5-diol ++: Reduced growth compared to the control +: Very poor growth -: No growth | |

**Table 2. MIC of chlorhexidine digluconate against S. aureus**

| Chlorhexidine (wt %) | MIC |
|---|---|
| 0,00001 | +++ |
| 0,00005 | ++ |
| 0,0001 | - |
| 0,0005 | - |
| 0,001 | - |
| 0,005 | - |
| 0,01 | - |
| 0,02 | - |
| 0,05 | - |
| 0,1 | - |
| 0,5 | - |
| 1 | - |
| Control (no chlorhexidine) | +++ |

### Example 2, MIC of pentane-1,5-diol in combination with chlorhexidine against S. aureus

The minimal inhibitory concentration (MIC) of pentane-1,5-diol combined with chlorhexidine against *S. aureus* was determined using bacteria concentrations of 6 x 10⁶ (20 µl applicated onto DST-agar plates). The results are depicted below in table 3.

**Table 3. MIC of pentane-1,5-diol combined with chlorhexidine digluconate against S. aureus**

| Chlorhexidine 0,0001% and | MIC | Chlorhexidine 0,00005% and | MIC | Chlorhexidine 0,00001% and | MIC |
|---|---|---|---|---|---|
| 9% Pentane-1,5-diol | - | 9% Pentane-1,5-diol | - | 9% Pentane-1,5-diol | - |
| 8% Pentane-1,5-diol | - | 8% Pentane-1,5-diol | - | 8% Pentane-1,5-diol | - |
| 7% Pentane-1,5-diol | - | 7% Pentane-1,5-diol | - | 7% Pentane-1,5-diol | + |
| 6% Pentane-1,5-diol | - | 6% Pentane-1,5-diol | - | 6% Pentane-1,5-diol | ++ |
| 5% Pentane-1,5-diol | - | 5% Pentane-1,5-diol | - | 5% Pentane-1,5-diol | +++ |

As seen from table 1-3 there is clearly a synergistic antibacterial effect in combining pentane-1,5-diol with chlorhexidine digluconate. While neither chlorhexidine in concentrations at or below 0,00005% nor pentane-1,5-diol in concentrations at or below 8 % is effective agains *S. aureus*, a combination of 0,00005 % chlorhexidine with 5% or more of pentane-1,5-diol is. Further, even such low concentrations of chlorhexidine and pentane-1,5-diol as 0,00001 % and 7 %, respectively, are effective when combined.

### Example 3, MIC of pentane-1,5-diol and chlorhexidine against P. acnes

The minimal inhibitory concentration (MIC) of pentane-1,5-diol and chlorhexidine against *P. acnes* was determined using bacteria concentrations of 6 x 10⁶ (20 µl applicated onto blood agar plates). The results are depicted below in table 4 and 5.

**Table 4. MIC of pentane-1,5-diol against P. acnes**

| Pentane-1,5-diol (wt %) | MIC |
|---|---|
| 1 | +++ |
| 3 | ++ |
| 5 | + |
| 6 | - |
| 7 | - |
| 10 | - |
| 20 | - |
| 30 | - |
| Control (no pentane-1,5-diol) | +++ |

**Table 5. MIC of chlorhexidine digluconate against P. acnes**

| Chlorhexidine (wt %) | MIC |
|---|---|
| 0,00001 | +++ |
| 0,00005 | ++ |
| 0,0001 | - |
| 0,0005 | - |
| 0,001 | - |
| 0,005 | - |
| 0,01 | - |
| 0,02 | - |
| 0,05 | - |
| 0,1 | - |
| 0,5 | - |
| 1 | - |
| Control (no chlorhexidine) | +++ |

### Example 4, MIC of pentane-1,5-diol in combination with chlorhexidine against P. acnes

The minimal inhibitory concentration (MIC) of pentane-1,5-diol combined with chlorhexidine against *P. acnes* was determined using bacteria concentrations of 6 x 10⁶ (20 µl applicated onto blood agar plates). The results are depicted below in table 6.

**Table 6. MIC of pentane-1,5-diol combined with chlorhexidine digluconate against P. acnes**

| Chlorhexidine 0,0001% and | MIC | Chlorhexidine 0,00005% and | MIC | Chlorhexidine 0,00001% and | MIC |
|---|---|---|---|---|---|
| 6% Pentane-1,5-diol | - | 6% Pentane-1,5-diol | - | 6% Pentane-1,5-diol | + |
| 5% Pentane-1,5-diol | - | 5% Pentane-1,5-diol | - | 5% Pentane-1,5-diol | ++ |
| 3% Pentane-1,5-diol | - | 3% Pentane-1,5-diol | - | 3% Pentane-1,5-diol | +++ |
| 1% Pentane-1,5-diol | - | 1% Pentane-1,5-diol | ++ | 1% Pentane-1,5-diol | +++ |

As seen from table 4-6 there is clearly a synergistic antibacterial effect in combining pentane-1,5-diol with chlorhexidine digluconate. While neither chlorhexidine in concentrations at or below 0,00005% nor pentane-1,5-diol in concentrations at or below 5 % is effective agains *P. acnes*, a combination of 0,00005 % chlorhexidine with 3% or more of pentane-1,5-diol is.

### Example 5, MIC of pentane-1,5-diol in combination with chlorhexidine against S. pyogenes A CCUG 4208

The minimal inhibitory concentration (MIC) of pentane-1,5-diol combined with chlorhexidine against *S. pyogenes* A CCUG 4208 was determined using bacteria concentrations of 6 x 10⁷ (20 µl applicated onto blood agar plates). The results are depicted below in table 7 and 8.

**Table 7. MIC of chlorhexidine digluconate and 1,5-pentanediol against S. pyogenes**

| 1,5-pentanediol (%) | MIC | **chlorhexidine digluconate (%)** | MIC |
|---|---|---|---|
| 2 | +++ | 0.00001 | +++ |
| 4 | ++ | 0.00005 | +++ |
| 6 | + | 0.0001 | + |
| 7 | - | 0.0005 | - |
| 8 | - | 0.001 | - |
| 9 | - | 0.005 | - |
| 10 | - | 0.01 | - |
| 15 | - | 0.05 | - |
| | | 0.1 | - |
| | | 0.5 | - |

The positive control (agar plate without substance) showed a MIC value corresponding to +++.

**Table 8. MIC of pentane-1,5-diol combined with chlorhexidine digluconate against S. pyogenes**

| 1,5-pentandiol (2%) | | MIC |
|---|---|---|
| #1 Chlorhexidinne digluconate | 0,00001% | ++ |
| #2 Chlorhexidinne digluconate | 0,00005% | + |
| #3 Chlorhexidinne digluconate | 0,0001 % | - |
| | | |

| 1,5-pentandiol (4%) | | MIC |
|---|---|---|
| #4 Chlorhexidine digluconate | 0,00001% | + |
| #5 Chlorhexidinne digluconate | 0,00005% | - |
| #6 Chlorhexidinne digluconate | 0,0001 | - |
| | | |

| 1,5-pentandiol (6%) | | MIC |
|---|---|---|
| #7 Chlorhexidine digluconate | 0,00001% | - |
| #8 Chlorhexidinne digluconate | 0,00005% | - |
| #9 Chlorhexidinne digluconate | 0,0001% | - |

As seen from table 7 and 8 there is clearly a synergistic antibacterial effect in combining pentane-1,5-diol with chlorhexidine digluconate. While neither chlorhexidine in concentrations at or below 0,0005% nor pentane-1,5-diol in concentrations at or below 6 % is effective agains *S. pyogenes*, a combination of 0,00001 % chlorhexidine with 4% or more of pentane-1,5-diol is.

### Example 6 MIC of pentane-1,5-diol in combination with chlorhexidine

Chlorhexidine digluconate (CHG) solution, 20% water, C9394-25ML, (Sigma, St. Louis, USA) and PD, (BASF AG, Ludwigshafen, Germany) were used in the study and the following reference strains were tested; C. *albicans* (CCUG 5594), *S. aureus* (CCUG 17621) and *P*. *acnes* (CCUG 1794).

Minimum inhibitory concentration of CHG and pentane-1,5-diol (PD) were determined by agar dilution assay. A series of dilutions of each compound were prepared in blood agar medium for *P. acnes*, and in Diagnostic Sensitivity Test (DST) agar (Oxoid, Basingstoke, UK) for C. *albicans* and *S. aureus*. Dilution series with each agent were made with freshly prepared agar at 48-50°C. Final concentration ranges were as follows: CHG solution, 0.00001 to 1 % (w/v) and PD, 1-30% (v/v). The prepared plates were solidified and used immediately.

For each organism an inoculum was prepared by growing organisms on blood agar. Colonies were suspended in phosphate-buffered saline (PBS) and suspensions were adjusted to approximately 6x10⁶ CFU/ml for *C*. *albicans* and *P. acnes* and 6x10⁵ CFU/mL for *S. aereus*. The plates were inoculated with 20 µl spots and incubated for one day for C. *albicans* and *S. aureus* and three days for *P. acnes*. MICs were then determined as the lowest concentration of the compound inhibiting growth of the isolates.

First the MICs of CHG and PD, respectively, were determined for each micro-organism. Then, a range of combined CHG and PD concentrations were tested on each micro-organism to determine if there was synergistic activity. The definition of synergistic effect is: "An effect that is greater than the sum of the effects of the two drugs, such as the equation: 1+1...". Each isolate was tested twice.

CHG was effective against all micro-organisms (Table 9). MIC's of CHG were 0.0005 (% w/v) against *C. albicans* and 0.0001 (% w/v) against *S. aureus* and *P. acnes*.

PD demonstrated an activity against C. *albicans*, *S. aureus* and *P. acnes* of 5, 9 and 6, % v/v, respectively. MIC of CHG was much lower than PD with a factor of 1x10⁻⁴ for *C. albicans*, 6x10⁻⁵ for *S. aureus* and 9x10⁻⁵ for *P. acnes* (Table 9).

When CHG and PD were combined, in concentrations lower than their respective MICs, their antimicrobial activity increased. MICs of CHG was reduced from 0.0005 to 0.00005 (%, w/v) and MIC's of PD from 5 to 3 (%, v/v) for totally preventing growth of C. *albicans*. MICs of CHG was reduced from 0.0001 to 0.00001 (%, w/v) and MIC's of PD from 9 to 8 (%, v/v) for *S. aureus*. MIC's of CHG was reduced from 0.0001 to 0.00005 (%, w/v) and MIC's of PD from 6 to 3 (%, v/v) for totally preventing growth of *P. acnes* (Table 9).

**Table 9. Minimum inhibitory concentration (MIC) of chlohexidine digluconate (CHG) (w/v %) and pentane-1,5-diol (PD) (v/v %) alone and in combination against C. albicans, S. aureus and P. acnes by agar dilution method. Ratio indicates reduction of the MIC of CHG and PD alone and in combination.**

| Strains | MIC alone | | MIC combined | Ratio (alone/combined) | |
|---|---|---|---|---|---|
| | CHG | PD | CHG PD | CHG/CHG+PD | PD/PD+CHG |
| *C. albicans* | 0.0005 | 5 | 0.00005 3 | 10 | 2 |
| CCUG5594 | | | | | |
| *S. aureus* | 0.0001 | 9 | 0.00001 8 | 10 | 1 |
| CCUG17621 | | | | | |
| *P. acnes* | 0.0001 | 6 | 0.00005 3 | 2 | 2 |
| CCUG1794 | | | | | |

## Claims

1. An antimicrobial composition comprising chlorhexidine and pentane-1,5-diol, wherein the amount of chlorhexidine is between 0.000001 and 5 wt % and the amount of pentane-1,5-diol is between 1 and 75 wt %.

2. The composition according to claim 1, wherein the amount of chlorhexidine is between 0.00001 and 5 wt % and the amount of pentane-1,5-diol is between 3 and 25 wt%.

3. The composition according to claim 2, wherein the amount of chlorhexidine is between 0.001 and 2.5 wt % and the amount of pentane-1,5-diol is between 5 and 15 wt%.

4. The composition according to any of the claims 1 to 3, wherein said composition is a pharmaceutical composition.

5. The composition according to claim 4, wherein said composition is for topical administration.

6. The composition according to claim 4 or 5, wherein said composition comprises a carrier and a thickening agent.

7. The composition according to any of the preceding claims for use in therapy.

8. Use of a composition according to any of the claims 1 to 3 in non-therapeutic disinfection.

9. The composition according to any of the claims 1 to 6 for use in treating infections.

10. The composition for use according to claim 9, wherein said infection is an infection affecting the skin.

## Patentansprüche

1. Antimikrobielle Zusammensetzung umfassend Chlorhexidin und Pentan-1,5-diol, wobei die Menge an Chlorhexidin zwischen 0,000001 und 5 Gewichts-% beträgt, und die Menge an Pentan-1,5-diol zwischen 1 und 75 Gewichts-% beträgt.

2. Zusammensetzung nach Anspruch 1, wobei die Menge an Chlorhexidin zwischen 0,00001 und 5 Gewichts-% beträgt, und die Menge an Pentan-1,5-diol zwischen 3 und 25 Gewichts-% beträgt

3. Zusammensetzung nach Anspruch 2, wobei die Menge an Chlorhexidin zwischen 0,001 und 2,5 Gewichts-% beträgt, und die Menge an Pentan-1,5-diol zwischen 5 und 15 Gewichts-% beträgt.

4. Zusammensetzung nach einem beliebigen der Ansprüche 1 bis 3, wobei die Zusammensetzung eine pharmazeutische Zusammensetzung ist.

5. Zusammensetzung nach Anspruch 4, wobei die Zusammensetzung zur topischen Verabreichung ist.

6. Zusammensetzung nach Anspruch 4 oder 5, wobei die Zusammensetzung einen Träger und ein Verdickungsmittel umfasst.

7. Zusammensetzung nach einem beliebigen der voranstehenden Ansprüche zur Verwendung in der Therapie.

8. Verwendung einer Zusammensetzung nach einem beliebigen der Ansprüche 1 bis 3 in nicht-therapeutischer Desinfektion.

9. Zusammensetzung nach einem beliebigen der Ansprüche 1 bis 6 zur Verwendung beim Behandeln von Infektionen.

10. Zusammensetzung zur Verwendung nach Anspruch 9, wobei die Infektion eine Infektion ist, die die Haut beeinträchtigt.

## Revendications

1. Composition antimicrobienne comprenant de la chlorhexidine et du pentane-1,5-diol, dans laquelle la quantité de chlorhexidine est comprise entre 0,000001 et 5 % en poids et la quantité de pentane-1,5-diol est comprise entre 1 et 75 % en poids.

2. Composition selon la revendication 1, dans laquelle la quantité de chlorhexidine est comprise entre 0,00001 et 5 % en poids et la quantité de pentane-1,5-diol est comprise entre 3 et 25 % en poids.

3. Composition selon la revendication 2, dans laquelle la quantité de chlorhexidine est comprise entre 0,001 et 2,5 % en poids et la quantité de pentane-1,5-diol est comprise entre 5 et 15 % en poids.

4. Composition selon l'une quelconque des revendications 1 à 3, dans laquelle ladite composition est une composition pharmaceutique.

5. Composition selon la revendication 4, dans laquelle ladite composition est destinée à une administration topique.

6. Composition selon la revendication 4 ou 5, dans laquelle ladite composition comprend un support et un agent épaississant.

7. Composition selon l'une quelconque des revendications précédentes destinée à une utilisation en thérapie.

8. Utilisation d'une composition selon l'une quelconque des revendications 1 à 3, dans une désinfection non thérapeutique.

9. Composition selon l'une quelconque des revendications 1 à 6, destinée à une utilisation dans le traitement d'infections.

10. Composition destinée à une utilisation selon la revendication 9, dans laquelle ladite infection est une infection affectant la peau.
